# EUROPÄISCHE PATENTSCHRIFT

(11) **EP 2 603 484 B1**
(45) Veröffentlichungstag und Bekanntmachung des Hinweises auf die Patenterteilung: **14.01.2015**
(21) Anmeldenummer: 11748572.2
(22) Anmeldetag: 06.08.2011
(51) Int. Cl.: C07C 67/14, C07C 69/24, C11D 3/39

(54) **VERFAHREN ZUR HERSTELLUNG VON ACYLOXYBENZOESÄUREN**
METHOD FOR PRODUCING ACYLOXY BENZOIC ACIDS
PROCÉDÉ DE PRÉPARATION D'ACIDES ACYLOXYBENZOÏQUES

(30) Priorität: 13.08.2010 DE 102010034244
(43) Veröffentlichungstag der Anmeldung: 19.06.2013
(73) Patentinhaber: WeylChem Switzerland AG, 4132 Muttenz (CH)
(72) Erfinder: SAJITZ, Melanie, 58840 Plettenberg (DE); SCHEFFER, Isabel, 65929 Frankfurt (DE); JANITSCHEK, Werner, 65558 Heistenbach (DE)
(74) Vertreter: Ackermann, Joachim
(86) Internationale Anmeldenummer: PCT/EP2011/003956
(87) Internationale Veröffentlichungsnummer: WO 2012/019742

(56) Entgegenhaltungen:
- WO-A2-2004/002979
- US-A- 5 891 838

## Beschreibung

Die vorliegende Erfindung betrifft ein Verfahren zur Herstellung von Acyloxybenzoesäuren ausgehend von para-Hydroxybenzoesäure und Carbonsäurehalogeniden.

Anorganische Persauerstoffverbindungen, insbesondere Wasserstoffperoxid und feste Persauerstoffverbindungen, die sich in Wasser unter Freisetzung von Wasserstoffperoxid lösen, wie Natriumperborat und Natriumpercarbonat, werden seit langem als Oxidationsmittel zu Desinfektions- und Bleichzwecken verwendet. Die Oxidationswirkung dieser Substanzen hängt in verdünnten Lösungen stark von der Temperatur ab. So erzielt man beispielsweise mit H₂O₂ oder Perborat in alkalischen Bleichflotten erst bei Temperaturen oberhalb von etwa 80 °C eine ausreichend schnelle Bleiche verschmutzter Textilien.

Bei niedrigeren Temperaturen kann die Oxidationswirkung der anorganischen Persauerstoffverbindungen durch Zusatz so genannter Bleichaktivatoren verbessert werden. Hierfür wurden in der Vergangenheit zahlreiche Vorschläge erarbeitet, vor allem aus den Stoffklassen der N- oder O-Acylverbindungen.

In den letzten Jahren waren die Verbindungen gemäß der Formel (la) 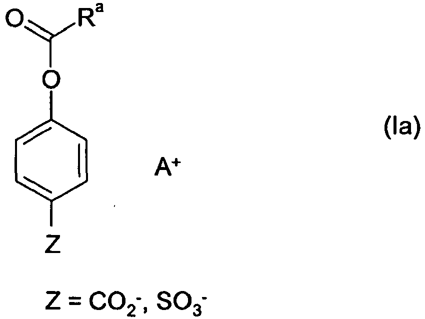 worin der Rest R^{a} insbesondere für eine lineare oder verzweigte, gesättigte Alkylgruppe mit 6 bis 22 Kohlenstoffatomen oder für eine lineare oder verzweigte, ein- oder mehrfach ungesättigte Alkenylgruppe mit 6 bis 22 Kohlenstoffatomen steht und

A⁺ insbesondere ein Alkali- oder Erdalkalimetallion und vorzugsweise ein Natriumion bedeutet, von Interesse als Aktivatoren für anorganische Peroxyverbindungen. Insbesondere war die Verwendung dieser Verbindungen als Bleichmittel bzw. als Peroxysäure-Vorstufe von Interesse.

Diese Peroxysäure-Vorstufen reagieren in wässriger Lösung mit den anorganischen Peroxyverbindungen wie Natriumpercarbonat oder Natriumperborat, die im Waschmittel enthalten sind, und bilden organische Peroxysäuren, die bei niedrigen Temperaturen (< 70 °C) viel effektiver bleichen als die anorganischen Peroxyverbindungen.

In besonderer Weise zeichnen sich Acyloxybenzoesäuren gemäß der Formel (Ib) 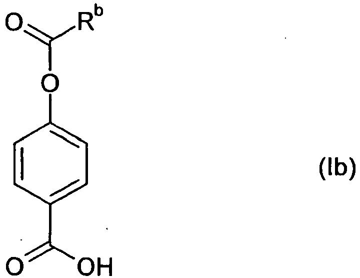 worin R^{b} insbesondere eine lineare oder verzweigte, gesättigte Alkylgruppe mit 6 bis 30 Kohlenstoffatomen, eine lineare oder verzweigte, ein- oder mehrfach ungesättigte Alkenylgruppe mit 6 bis 30 Kohlenstoffatomen, oder eine Arylgruppe mit 6 bis 30 Kohlenstoffatomen ist, sowie auch deren Salze, bei denen es sich insbesondere um Alkali- oder Erdalkalimetallsalze handelt aus, da sie nicht nur sehr gute Bleichaktivatoren sind, sondern sich vorteilhafter Weise nicht als hautsensibilisierend erweisen.

Sowohl die Phenolestersulfonate als auch die Acyloxybenzoesäuren und deren Salze sind prinzipiell z. B. durch Umsetzung von Carbonsäurechloriden wie beispielsweise Alkylsäurechlorid mit Phenolsulfonat oder para-Hydroxybenzoesäure nach der Schotten-Baumann-Reaktion zugänglich. Beispiele hierfür finden sich u. a. in EP 0 294 073, EP 0 164 786 oder WO 92/15556.

Die Acyloxybenzoesäuren können aus Säurechloriden und para-Hydroxybenzoesäure auch bei hohen Temperaturen > 30 °C hergestellt werden (JP 4194688 oder WO 2004/002979). Ein Nachteil des Verfahrens ist jedoch die Bildung von Nebenkomponenten wie Dimere und Trimere der para-Hydroxybenzoesäure bzw. Estern der dimeren para-Hydroxybenzoesäure. Das Vorkommen derartiger Nebenkomponenten, die sich nicht abtrennen lassen, ist in Waschmitteln äußerst unerwünscht.

In US 5,891,838 wird ein Verfahren zur Herstellung von para-Decanoyloxybenzoesäure offenbart, das als Lösungsmittelgemisch Diethylether und Wasser vorsieht. Die Ausbeute von 40 % ist allerdings unbefriedigend und die großtechnische Handhabung von Diethylether aus Arbeitsschutzgründen unerwünscht.

Aufgabe der vorliegenden Erfindung war es, ein Verfahren zur Herstellung von langkettigen oder aromatischen Acyloxybenzoesäuren zur Verfügung zu stellen, das großtechnisch durchführbar ist, in hohen Ausbeuten zu den Acyloxybenzoesäuren führt, und wobei die Acyloxybenzoesäuren hinsichtlich Zusammensetzung und Qualität für den Einsatz in Wasch- und Reinigungsmitteln geeignet sind.

Es wurde nun gefunden, dass diese Aufgabe gelöst wird durch ein Verfahren zur Herstellung von Acyloxybenzoesäuren der Formel (I) 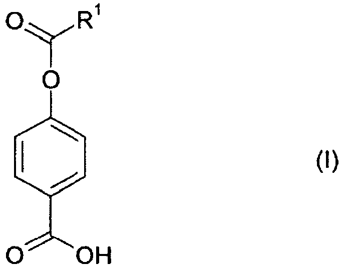 worin
- R¹: eine lineare oder verzweigte, gesättigte Alkylgruppe mit 6 bis 30 Kohlenstoffatomen, eine lineare oder verzweigte, ein- oder mehrfach ungesättigte Alkenylgruppe mit 6 bis 30 Kohlenstoffatomen, oder eine Arylgruppe mit 6 bis 30 Kohlenstoffatomen ist,
welches dadurch gekennzeichnet ist, dass
a) ein Carbonsäurehalogenid der Formel R¹COHal, worin R¹ die oben angegebene Bedeutung besitzt und Hal ein Halogenid ist, mit para-Hydroxybenzoesäure in Gegenwart von Base, wobei die eingesetzte Base ausgewählt ist aus Alkalihydroxiden, in einem Lösungsmittelgemisch enthaltend Wasser und ein organisches Lösungsmittel, wobei das eingesetzte organische Lösungsmittel iso-Propanol ist, bei einer Temperatur ≤ 25 °C und einem pH-Wert von 9 bis 11,5 umgesetzt wird,
b) das Reaktionsgemisch nach Schritt a) bei einer Temperatur ≤ 25 °C auf pH 6 bis 8 durch Zugabe von Säure eingestellt wird und
c) das Reaktionsgemisch nach Schritt b) auf eine Temperatur von 35 bis 80 °C erwärmt und danach durch Zugabe von Säure auf pH 1 bis 4 eingestellt wird.

Gegenstand der Erfindung ist daher ein Verfahren zur Herstellung von Acyloxybenzoesäuren der Formel (I) 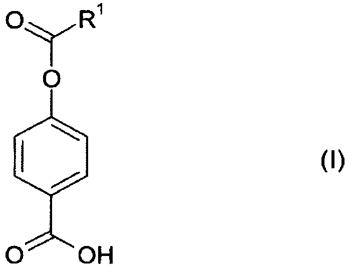 worin
- R¹: eine lineare oder verzweigte, gesättigte Alkylgruppe mit 6 bis 30, vorzugsweise 7 bis 15 und besonders bevorzugt 7 bis 11 Kohlenstoffatomen, eine lineare oder verzweigte, ein- oder mehrfach ungesättigte Alkenylgruppe mit 6 bis 30, vorzugsweise 7 bis 15 und besonders bevorzugt 7 bis 11 Kohlenstoffatomen, oder eine Arylgruppe mit 6 bis 30 Kohlenstoffatomen ist,
dadurch gekennzeichnet, dass
a) ein Carbonsäurehalogenid der Formel R¹COHal, worin R¹ die oben angegebene Bedeutung besitzt und Hal ein Halogenid ist, mit para-Hydroxybenzoesäure in Gegenwart von Base, wobei die eingesetzte Base ausgewählt ist aus Alkalihydroxiden, in einem Lösungsmittelgemisch enthaltend Wasser und ein organisches Lösungsmittel, wobei das eingesetzte organische Lösungsmittel iso-Propanol ist, bei einer Temperatur ≤ 25 °C und vorzugsweise von 0 bis 25 °C und einem pH-Wert von 9 bis 11,5, vorzugsweise von 10 bis 11 und besonders bevorzugt von 10,3 bis 10,7, umgesetzt wird,
b) das Reaktionsgemisch nach Schritt a) bei einer Temperatur ≤ 25 °C und vorzugsweise von 0 bis 25 °C auf pH 6 bis 8 durch Zugabe von Säure eingestellt wird und
c) das Reaktionsgemisch nach Schritt b) auf eine Temperatur von 35 bis 80 °C, vorzugsweise von 50 bis 75 °C, erwärmt und danach durch Zugabe von Säure, vorzugsweise HCl, auf pH 1 bis 4 eingestellt wird.

Das erfindungsgemäße Verfahren besitzt den Vorteil, dass die Acyloxybenzoesäuren der Formel (I) in einfacher Weise aus dem Reaktionsgemisch abfiltriert werden können. Die nach dem erfindungsgemäßen Verfahren erhaltenen Acyloxybenzoesäuren sind weiß bis cremefarben und können in Form von Pulver oder Granulat zur Herstellung von Wasch- und Reinigungsmittel eingesetzt werden. Besonders vorteilhaft ist, dass die nach dem erfindungsgemäßen Verfahren erhaltenen Acyloxybenzoesäuren eine sehr hohe Reinheit aufweisen und Nebenkomponenten wie z. B. Dimere oder Trimere der para-Hydroxybenzoesäure vorzugsweise in einer Menge < 1,0 Gew.-%, besonders bevorzugt in einer Menge < 0,3 Gew.-% und insbesondere bevorzugt in einer Menge < 0,1 Gew.-% enthalten. Im Gegensatz zu Acyloxybenzoesäuren aus anderen Verfahren, die in der Regel Nebenprodukte enthalten, sind die Acyloxybenzoesäuren aus dem erfindungsgemäßen Verfahren aufgrund ihrer hohen Reinheit besonders gut geeignet, in Wasch- und Reinigungsmitteln eingesetzt zu werden.

In dem erfindungsgemäßen Verfahren wird nicht die gesamte Menge der in Schritt a) verwendeten Base vor der Zugabe des Carbonsäurehalogenids in den Reaktionsansatz gegeben. Vielmehr wird die Base im Verlauf des Schrittes a) so zudosiert, dass der pH-Wert während der Umsetzung bzw. der Reaktion des Carbonsäurehalogenids mit dem aus der para-Hydroxybenzoesäure hervorgehenden Phenolat in Schritt a) in dem pH-Wert-Bereich von 9 bis 11,5, vorzugsweise von 10 bis 11 und besonders bevorzugt von 10,3 bis 10,7, gehalten wird.

In Schritt a) des erfindungsgemäßen Verfahrens wird vorzugsweise so vorgegangen, dass das Carbonsäurehalogenid der Formel R¹COHal zu einer Mischung aus para-Hydroxybenzoesäure und Alkalihydroxid in einem Lösungsmittelgemisch enthaltend Wasser und iso-Propanol zugegeben wird. Hierbei wird anfangs jedoch nur soviel Alkalihydroxid in den Reaktionsansatz gegeben, dass ein Anfangs-pH-Wert von 9 bis 11,5, vorzugsweise von 10 bis 11 und besonders bevorzugt von 10,3 bis 10,7, erreicht wird. Da bei der Umsetzung in Schritt a) Halogenwasserstoff HHal, wobei Hal die oben angegebene Bedeutung besitzt, gebildet wird, wird der pH-Wert des Reaktionsgemisches im Reaktionsverlauf erniedrigt. Durch Zugabe von weiterem Alkalihydroxid wird der pH-Wert während des gesamten Schrittes a) in dem oben genannten Bereich von 9 bis 11,5, vorzugsweise von 10 bis 11 und besonders bevorzugt von 10,3 bis 10,7, gehalten.

Bevorzugte Carbonsäurehalogenide der Formel R¹COHal sind diejenigen, worin Hal Cl, Br oder I ist. Besonders bevorzugte Carbonsäurehalogenide der Formel R¹COHal sind diejenigen, worin Hal Cl oder Br ist. Insbesondere bevorzugte Carbonsäurehalogenide der Formel R¹COHal sind die Carbonsäurechloride gemäß der Formel (II)

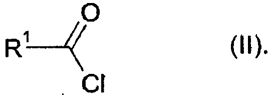

In dem Fall, dass der Rest R¹ eine Arylgruppe darstellt, ist er vorzugsweise eine Phenylgruppe oder eine Phenylgruppe, die mit 1 bis 3 Methylgruppen substituiert ist. Unter diesen substituierten Phenylgruppen ist die Gruppe -C₄H₆-CH₃ wiederum bevorzugt. Unter den Arylgruppen ist jedoch die (unsubstituierte) Phenylgruppe besonders bevorzugt.

Vorzugsweise ist der Rest R¹ jedoch eine Alkyl- oder Alkenylgruppe. Beispiele für den Carbonsäurehalogeniden R¹-COHal zugrunde liegende Carbonsäuren R¹-COOH sind Heptansäure, Octansäure, Methyloctansäure, Nonansäure, 3,3,5-Isononansäure, Decansäure, Undecansäure, Undecensäure, Dodecansäure, Tetradecansäure, gehärtete Talgfettsäure und Octadecansäure.

Besonders bevorzugt ist der Rest R¹ eine Alkylgruppe. Insbesondere bevorzugt sind die den Carbonsäurehalogeniden zugrunde liegenden Carbonsäuren R¹-COOH ausgewählt aus der Gruppe bestehend aus Octansäure, Nonansäure, 3,3,5-Isononansäure, Decansäure und Dodecansäure. Unter diesen sind wiederum Nonansäure und Decansäure bevorzugt und Decansäure ist besonders bevorzugt.

Vorzugsweise wird das erfindungsgemäße Verfahren derart ausgeführt, dass nach Schritt c) das Reaktionsgemisch auf eine Temperatur < 35 °C, besonders bevorzugt < 30 °C, insbesondere bevorzugt von 0 bis 30 °C und außerordentlich bevorzugt von 5 bis 25 °C abgekühlt wird (Schritt d)). Hierdurch kann insbesondere eine Ausbeutesteigerung erzielt werden.

Vorzugsweise liegen die nach dem erfindungsgemäßen Verfahren hergestellten Acyloxybenzoesäuren der Formel (I) in Form von Partikeln mit d₅₀-Werten von 10 bis 150 µm vor. Unter diesen Partikeln sind wiederum diejenigen bevorzugt, die d₁₀-Werte von 5 bis 30 µm und d₉₀-Werte von 30 bis 200 µm besitzen.

Besonders bevorzugt liegen die nach dem erfindungsgemäßen Verfahren hergestellten Acyloxybenzoesäuren der Formel (I) in Form von Partikeln mit d₅₀-Werten von 12 bis 100 µm vor. Unter diesen Partikeln sind wiederum diejenigen bevorzugt, die d₁₀-Werte von 6 bis 25 µm und d₉₀-Werte von 40 bis 150 µm besitzen.

Die d₅₀-Werte der nach dem erfindungsgemäßen Verfahren hergestellten Partikel sind im Rahmen der vorliegenden Anmeldung wie folgt definiert: ein d₅₀-Wert von "x" µm bedeutet, dass genau 50 Volumen-% der Partikel einen Durchmesser kleiner als "x" µm besitzen. Die analoge Definition gilt auch für die d₁₀- und die d₉₀-Werte.

Die angegebenen d₁₀-, d₅₀- und d₉₀-Werte sind Werte für die nach dem erfindungsgemäßen Verfahren hergestellten Acyloxybenzoesäuren der Formel (I) ohne nachträgliche Behandlung wie beispielsweise Siebung oder Mahlung, d. h. sie stellen Werte dar für direkt aus dem erfindungsgemäßen Verfahren erhaltene Acyloxybenzoesäuren der Formel (I).

Die Messung der Partikelgrößen der nach dem erfindungsgemäßen Verfahren hergestellten Acyloxybenzoesäuren der Formel (I) sowie ihrer d₁₀-, d₅₀- und d₉₀-Werte findet nach der Methode der Laser-Diffraktion (Laserbeugung) statt. Es wurde ein Gerät "Mastersizer 2000" mit einer "Scirocco 2000 dispersing unit" der Firma "Malvern Instruments" verwendet. Die "Scirocco 2000 dispersing unit" arbeitet auf Basis Luftdruck. Die Messung fand bei Raumtemperatur (25 °C) unter Verwendung von 2 g der Probe statt. Die Auswertung erfolgte über die Fraunhofer-Theorie (general purpose model).

Die bei der Acylierungsreaktion in Schritt a) des erfindungsgemäßen Verfahrens zugesetzte Base hat die Aufgabe, durch Bildung des Phenolats die Reaktion zu ermöglichen und den freiwerdenden Halogenwasserstoff HHal als Salz zu binden. Die in Schritt a) des erfindungsgemäßen Verfahrens eingesetzte Base ist ausgewählt aus Alkalihydroxiden. Besonders bevorzugt ist die in Schritt a) des erfindungsgemäßen Verfahrens eingesetzte Base KOH oder NaOH.

Das in Schritt a) des erfindungsgemäßen Verfahrens eingesetzte organische Lösungsmittel ist iso-Propanol.

Vorzugsweise besitzt die in Schritt b) und c) des erfindungsgemäßen Verfahrens eingesetzte Säure einen pKa-Wert kleiner oder gleich 4,0. Besonders bevorzugt ist diese Säure H₂SO₄ oder HCl und insbesondere bevorzugt HCI.

Vorzugsweise ist das Gewichtsverhältnis von Wasser zu iso-Propanol in Schritt a) von 5 : 1 bis 1 : 5 und besonders bevorzugt von 3 : 1 bis 1 : 2.

Vorzugsweise ist das Gewichtsverhältnis von Wasser zu para-Hydroxybenzoesäure in Schritt a) von 2 : 1 bis 10 : 1 und besonders bevorzugt von 2 : 1 bis 6 : 1.

Vorzugsweise ist das molare Verhältnis des Carbonsäurehalogenids der Formel R¹COHal zu der para-Hydroxybenzoesäure von 0,75 : 1 bis 1,5 : 1, besonders bevorzugt von 0,9 : 1 bis 1,1 : 1 und insbesondere bevorzugt 1 : 1.

Vorzugsweise ist das molare Verhältnis der Base, die in Schritt a) des erfindungsgemäßen Verfahrens eingesetzt wird, zu para-Hydroxybenzoesäure von 2 : 1 bis 2,5 : 1. Bei den hier angegebenen Mengen an Base handelt es sich um die Gesamtmenge an Base, die in Schritt a) des erfindungsgemäßen Verfahrens eingesetzt wird.

Zur Isolierung und Aufreinigung der nach dem erfindungsgemäßen Verfahren hergestellten Acyloxybenzoesäuren der Formel (I) wird vorzugsweise wie folgt vorgegangen: Das Reaktionsgemisch wird über konventionelle Trennmethoden filtriert (Filterapparate), vorzugsweise bei Raumtemperatur, und der Rückstand so lange mit Wasser gewaschen, bis kein Salz mehr vorhanden ist. Die Filtration findet vorzugsweise nach Schritt d) statt. Die gebildete Acyloxybenzoesäure fällt in hohen Ausbeuten in Form eines weißen Pulvers an, das durch konventionelle Methoden getrocknet werden kann.

Das Endprodukt enthält allenfalls Spuren der Carbonsäure R¹-COOH. Nicht umgesetzte para-Hydroxybenzoesäure und Salze wie beispielsweise Natriumchlorid können durch Waschen mit Wasser vollständig aus dem Filterkuchen entfernt werden.

Das Produkt des erfindungsgemäßen Verfahrens kann in vorteilhafter Weise als Aktivator für Wasserstoffperoxid eingesetzt werden.

Die nach dem erfindungsgemäßen Verfahren erhältlichen Acyloxybenzoesäuren sind in ihrer Wirkung als Bleichaktivator signifikant effektiver als Acyloxybenzoesäuren, die nach herkömmlichen Verfahren hergestellt werden. Die Persäure-Freisetzung erfolgt bei den nach dem erfindungsgemäßen Verfahren erhältlichen Acyloxybenzoesäuren wesentlich früher als bei nach konventionellen Verfahren hergestellten Acyloxybenzoesäuren.

Die nach dem erfindungsgemäßen Verfahren erhältlichen Acyloxybenzoesäuren können als Persalzaktivatoren in flüssigen oder pulverförmigen Wasch- und Reinigungsmitteln wie pulverförmigen Vollwaschmitteln, Fleckensalzen oder pulverförmigen Maschinengeschirrspülmitteln eingesetzt werden. Zur Erhöhung der Lagerstabilität in diesen Formulierungen können sie, wie dem Fachmann bekannt ist, in eine granulare Form überführt werden. Durch die Aktivierung lässt sich beispielsweise in Wasch- und Reinigungsmitteln die Bleichleistung der anorganischen Peroxyverbindungen / Wasserstoffperoxid verbessern oder in Desinfektionsmitteln die Desinfektionsleistung steigern.

Die folgenden Beispiele sollen die Erfindung näher erläutern ohne sie jedoch darauf einzuschränken.

### Beispiele

### Beispiel 1: Synthese von para-Decanoyloxy-benzoesäure (DOBA)

69,1 g (0,5 mol) para-Hydroxybenzoesäure wurden zunächst in 200 ml Wasser und 300 ml Isopropanol gelöst und bei 20 bis 25 °C mit 87,4 g NaOH-Lösung (32 Gew.-%ige wässrige Lösung, 0,7 mol) auf pH 10,5 gestellt. Zu dieser Lösung wurden dann bei pH 10,5 innerhalb von drei Stunden 95,4 g (0,5 mol) Decansäurechlorid zudosiert. Der pH-Wert wurde mit 40,5 g NaOH-Lösung (32 Gew.-%ige wässrige Lösung, 0,324 mol) auf pH 10,5 und die Temperatur auf 20 bis 25 °C gehalten. Der Ansatz wurde eine Stunde nachgerührt. Anschließend wurde das Reaktionsgemisch bei 20 bis 25 °C mit 5,3 g HCl-Lösung (32 Gew.-%ige wässrige Lösung) auf pH 7 gestellt, die komplette Lösung auf 65 bis 70 °C erhitzt und dann mit 60 g HCl-Lösung (32 Gew.-%ige wässrige Lösung) auf pH 1,5 bis 3 gestellt. Das Reaktionsgemisch wurde auf Raumtemperatur (25 °C) gekühlt, der Feststoff über eine Saugnutsche abfiltriert und zehnfach mit 150 ml Wasser gewaschen. Nach dem Trocknen im Vakuum bei 100 °C betrug die Ausbeute 134,1 g (91,7 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Vergleichsbeispiel 1: Synthese von para-Decanoyloxy-benzoesäure (DOBA)

69,1 g (0,5 mol) para-Hydroxybenzoesäure wurden zunächst in 200 ml Wasser und 300 ml Isopropanol gelöst und bei 20 bis 25 °C mit 87,4 g NaOH-Lösung (32 Gew.-%ige wässrige Lösung, 0,7 mol) auf pH 10,5 gestellt. Zu dieser Lösung wurden dann bei pH 10,5 innerhalb von drei Stunden 95,4 g (0,5 mol) Decansäurechlorid zudosiert. Der pH-Wert wurde mit 40,5 g NaOH-Lösung (32 Gew.-%ige wässrige Lösung, 0,324 mol) auf pH 10,5 und die Temperatur auf 20 bis 25 °C gehalten. Der Ansatz wurde eine Stunde nachgerührt. Anschließend wurde das Reaktionsgemisch bei 20 bis 25 °C mit 65,3 g HCl-Lösung (32 Gew.-%ige wässrige Lösung) auf pH 1,5 bis 3 gestellt. Das Reaktionsgemisch wurde über eine Saugnutsche abfiltriert. Hierbei gab es erhebliche Probleme, da sich die Nutsche/Filtriereinheit aufgrund der Kristallgröße des Produktes zugesetzt hatte. Der Versuch musste an dieser Stelle abgebrochen werden. Dies erfolgt ebenfalls, wenn das Produkt bei 65 °C gefällt und das Reaktionsgemisch bei 35 bis 65 °C abfiltriert wird.

### Beispiel 2: Synthese von para-Decanoyloxy-benzoesäure (DOBA)

Das Verfahren entsprach Beispiel 1, jedoch wurde das Lösungsmittelgemisch im umgekehrten Mengenverhältnis verwendet. Es wurden 300 ml Wasser und 200 ml Isopropanol als Lösungsmittelgemisch eingesetzt. Die Ausbeute betrug 127,2 g (87,0 % der Theorie). Die Reinheit des Produkts ist > 99,9 Gew.-%.

### Beispiel 3: Synthese von para-Decanoyloxy-benzoesäure (DOBA)

Das Verfahren entsprach Beispiel 1, jedoch wurde bei einer Ansatzgröße von 0,5 mol die Temperatur während der Dosierung des Decansäurechlorides und des Nachrührens sowie bei der Zugabe der HCl-Lösung gemäß Schritt b) des erfindungsgemäßen Verfahrens auf 0 bis 5 °C gehalten. Die Ausbeute betrug 133,9 g (91,6 % der Theorie). Die Reinheit des Produkts ist > 99,9 Gew.-%.

### Beispiel 4: Synthese von para-Benzoyloxy-benzoesäure (BOBA)

69,1 g (0,5 mol) para-Hydroxybenzoesäure wurden zunächst in 200 ml Wasser und 300 ml Isopropanol gelöst und bei 20 bis 25 °C mit 87,4 g NaOH-Lösung (32 Gew.-%ige wässrige Lösung, 0,7 mol) auf pH 10,5 gestellt. Zu dieser Lösung wurden dann bei pH 10,5 innerhalb von drei Stunden 70,2 g (0,5 mol) Benzoylchlorid zudosiert. Der pH-Wert wurde mit 40,5 g NaOH-Lösung (32 Gew.-%ige wässrige Lösung, 0,324 mol) auf pH 10,5 und die Temperatur auf 20 bis 25 °C gehalten. Der Ansatz wurde eine Stunde nachgerührt. Anschließend wurde das Reaktionsgemisch bei 20 bis 25 °C mit 5,3 g HCl-Lösung (32 Gew.-%ige wässrige Lösung) auf pH 7 gestellt und bei 65 bis 70 °C mit 60 g HCl-Lösung (32 Gew.-%ige wässrige Lösung) auf pH 1,5 bis 3 gestellt. Das Reaktionsgemisch wurde auf Raumtemperatur (25 °C) gekühlt, der Feststoff über eine Saugnutsche abfiltriert und zehnfach mit 150 ml Wasser gewaschen. Nach dem Trocknen im Vakuum bei 100 °C betrug die Ausbeute 119,1 g (98,3 % der Theorie). Das Produkt war laut HPLC- und NMR-Messung frei von Benzoesäure und nicht umgesetzter para-Hydroxybenzoesäure. Die Reinheit des Produkts ist > 99,9 Gew.-%.

### Beispiel 5: Synthese von para-Nonanoyloxy-benzoesäure (NOBA)

69,1 g (0,5 mol) para-Hydroxybenzoesäure wurden zunächst in 200 ml Wasser und 300 ml Isopropanol gelöst und bei 20 bis 25 °C mit 87,4 g NaOH-Lösung (32 Gew.-%ige wässrige Lösung, 0,7 mol) auf pH 10,5 gestellt. Zu dieser Lösung wurden dann bei pH 10,5 innerhalb von drei Stunden 88,3 g (0,5 mol) Nonansäurechlorid zudosiert. Der pH-Wert wurde mit 40,5 g NaOH-Lösung (32 Gew.-%ige wässrige Lösung, 0,324 mol) auf pH 10,5 und die Temperatur auf 20 bis 25 °C gehalten. Der Ansatz wurde eine Stunde nachgerührt. Anschließend wurde das Reaktionsgemisch bei 20 bis 25 °C mit 5,3 g HCl-Lösung (32 Gew.-%ige wässrige Lösung) auf pH 7 gestellt und bei 65 bis 70 °C mit 60 g HCl-Lösung (32 Gew.-%ige wässrige Lösung) auf pH 1,5 bis 3 gestellt. Das Reaktionsgemisch wurde auf Raumtemperatur (25 °C) gekühlt, der Feststoff über eine Saugnutsche abfiltriert und zehnfach mit 150 ml Wasser gewaschen. Nach dem Trocknen im Vakuum bei 100 °C betrug die Ausbeute 121,3 g (87,2 % der Theorie). Das Produkt war laut HPLC- und NMR-Messung frei von Nonansäure und nicht umgesetzter para-Hydroxybenzoesäure. Die Reinheit des Produkts ist > 99,9 Gew.-%.

### Beispiel 6: Synthese von para-Decanoyloxy-benzoesäure (DOBA)

69,1 g (0,5 mol) para-Hydroxybenzoesäure wurden zunächst in 200 ml Wasser und 300 ml Isopropanol gelöst und bei 20 bis 25 °C mit 78,4 g KOH-Lösung (50 Gew.-%ige wässrige Lösung, 0,7 mol) auf pH 10,5 gestellt. Zu dieser Lösung wurden dann bei pH 10,5 innerhalb von drei Stunden 95,4 g (0,5 mol) Decansäurechlorid zudosiert. Der pH-Wert wurde mit 44,0 g KOH-Lösung (50 Gew.-%ige wässrige Lösung, 0,392 mol) auf pH 10,5 und die Temperatur auf 20 bis 25 °C gehalten. Der Ansatz wurde eine Stunde nachgerührt. Anschließend wurde das Reaktionsgemisch bei 20 bis 25 °C mit 10 g HCl-Lösung (32 Gew.-%ige wässrige Lösung) auf pH 8 gestellt und bei 65 bis 70 °C mit 55 g HCl-Lösung (32 Gew.-%ige wässrige Lösung) auf pH 1,5 bis 3 gestellt. Das Reaktionsgemisch wurde auf Raumtemperatur (25 °C) gekühlt, der Feststoff über eine Saugnutsche abfiltriert und zehnfach mit 150 ml Wasser gewaschen. Nach dem Trocknen im Vakuum bei 100 °C betrug die Ausbeute 131,5 g (90,0 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 7: Synthese von para-Decanoyloxy-benzoesäure (DOBA)

Das Verfahren entsprach Beispiel 6, doch die Dosierung des Decansäurechlorids, das Nachrühren sowie die Zugabe der HCl-Lösung gemäß Schritt b) des erfindungsgemäßen Verfahrens wurden bei 10 bis 15 °C durchgeführt. Die Ausbeute betrug 133,2 g (91,0 % der Theorie). Die Reinheit des Produkts ist > 99,9 Gew.-%.

### Vergleichsbeispiel 2: Synthese von para-Decanoyloxy-benzoesäure (DOBA)

116,3 g (0,61 mol) Decansäurechlorid wurden in 300 ml Xylol auf 125 °C geheizt und innerhalb von 6 Stunden portionsweise 69,1 g (0,5 mol) 4-Hydroxybenzoesäure eingetragen. Der Ansatz wurde 1 Stunde bei 125 °C nachgerührt, auf Raumtemperatur abgekühlt, abgesaugt und dreimal mit 45 ml Xylol gewaschen. Nach dem Trocknen im Vakuum bei 100 °C betrug die Ausbeute 108,4 g (74 % der Theorie). Das Produkt war laut HPLC- und NMR-Messung frei von Decansäure und nicht umgesetzter para-Hydroxybenzoesäure, aber enthielt 0,4 Gew.-% Nebenprodukte wie Dimere und Trimere der para-Hydroxybenzoesäure.

### Vergleichsbeispiel 3: Synthese von para-Decanoyloxy-benzoesäure (DOBA) nach US 5,891,838, Beispiel XV

DOBA wurde nach Beispiel XV der US 5,891,838 hergestellt. Nach der Filtration des Produkts (welches eine schmierige Konsistenz aufwies, wodurch wiederum die Filtration nachteiliger Weise sehr lange dauerte), wurde wie folgt aufgearbeitet: Der Rückstand der Filtration wurde mehrmals mit Wasser gewaschen und das Produkt bei 100 °C im Vakuum getrocknet. Die Ausbeute betrug 90,6 g. Das Produkt enthielt laut HPLC- und NMR-Messung 10,9 Gew.-% Decansäure, 30,4 Gew.-% DOBA und 41 Gew.-% para-Hydroxybenzoesäure. Anschließend wurde das Produkt wie in US 5,891,838 zur Reinigung umkristallisiert und für die Messung der Persäurekinetik herangezogen (siehe nachfolgendes Beispiel 8).

### Beispiel 8: Bestimmung der Persäurekinetik von para-Decanoyloxy-benzoesäure (DOBA) aus dem erfindungsgemäßen Beispiel 1 und den Vergleichsbeispielen 2 und 3

Die Persäurekinetik wurde mittels iodometrischer Titration mit Natriumthiosulfat-Lösung bestimmt.

Die Messung beruht darauf, dass para-Decanoyloxy-benzoesäure (DOBA) und Wasserstoffperoxid in wässriger Lösung zu Perdecansäure und para-Hydroxybenzoesäure reagieren (sofern anorganische Peroxide verwendet werden, reagieren diese in wässriger Lösung zu Wasserstoffperoxid). Die Reaktion zwischen DOBA und Wasserstoffperoxid erfolgt in verdünnter wässriger Lösung bei einem pH-Wert von 10 bis 11 bei 20 °C schnell und quantitativ. Die gebildete Perdecansäure kann dann neben dem im Überschuss vorhandenen Wasserstoffperoxid iodometrisch bestimmt werden. Die Perdecansäure ist wesentlich reaktiver als Wasserstoffperoxid und oxidiert in schwach saurem Medium und bei tiefer Temperatur mit zugegebenem Iodid I⁻ (z. B. zugegeben in der Form von Kaliumiodid) sofort zu Iod I₂. Das entstandene Iod kann dann mit Natriumthiosulfat titriert werden. Aus der gefundenen Menge an Iod kann dann die entsprechende Menge an Perdecansäure berechnet werden.

Im Einzelnen wurde wie folgt vorgegangen:
In einem 2 Liter-Becherglas wurden 1 Liter entsalztes Wasser von 20 °C vorgelegt und gerührt. Hierzu wurden 1,5 g Natriumpercarbonat und 8 g eines Standardwaschmittels ("IEC 60 456 Typ A*" der WFK Testgewebe GmbH) gegeben und 2 Minuten vorgelöst. Anschließend wurden 0,25 g des zu untersuchenden DOBA zugegeben. Nach 3 Minuten wurden 50 ml abpipettiert und in ein 250 ml Becherglas auf 50 g Eis aus entsalztem Wasser und 10 ml Essigsäure (20 Gew.-%ige wässrige Lösung) gegeben. Anschließend wurden 5 ml wässrige Kaliumiodid-Lösung (10 Gew.-%ige wässrige Lösung) zugegeben und mit Natriumthiosulfat-Lösung (0,01 molare wässrige Lösung) titriert.

Zur Titration wurden ein "Titrino DMS 716" oder "Basic 794" (Metrohm) mit einer 50er Wechseleinheit und Keyboard sowie ein "Ti Stand 727" (Metrohm) mit gezogener Bürettenspitze, Rührstab und kombinierter Platinelektrode verwendet.

Die nächsten Proben wurden nach bestimmten Zeiten nach der DOBA-Zugabe gezogen und wie oben beschrieben titriert. Die Menge an Perdecansäure nähert sich mit zunehmender Zeit der Probennahme einem Maximalwert an und bleibt dann bei noch später gezogenen Proben konstant. Dieser Maximalwert für die Menge an Perdecansäure wird auf 100 % gesetzt. Die Mengen an Perdecansäure für die anderen Proben werden dann in Relation zu diesen 100 % gesetzt.

Die Ergebnisse der Bestimmung der Persäurekinetik, d. h. die Ergebnisse der Bestimmung der Menge an Perdecansäure in Abhängigkeit von der Zeit, sind in Tabelle 1 gezeigt.

**Tabelle 1: Ergebnisse der Bestimmung der Persäurekinetik von para-Decanoyloxy-benzoesäure (DOBA)**

| Zeit [Min.] | Menge an Perdecansäure [%] | | |
|---|---|---|---|
| | Verwendung von DOBA aus erfindungsgemäßem Beispiel 1 | Verwendung von DOBA aus Vergleichsbeispiel 2 | Verwendung von DOBA aus Vergleichsbeispiel 3 |
| 3 | 89,83 | 66,85 | 82,04 |
| 6 | 96,39 | 82,92 | 84,28 |
| 9 | 98,53 | 87,98 | 88,00 |
| 12 | 100,00 | 92,91 | 93,38 |
| 15 | | 96,44 | 94,32 |
| 18 | | 100,00 | 100,00 |

Die Ergebnisse der Tabelle 1 beschreiben die Wirkstoff-Freisetzung aus para-Decanoyloxy-benzoesäure (DOBA), d. h. hier die Freisetzung von Perdecansäure, in Abhängigkeit von der Zeit. Sie lassen erkennen, dass der Wirkstoff aus dem DOBA, welches nach dem erfindungsgemäßen Beispiel 1 hergestellt worden ist, schneller freigesetzt wird, als der Wirkstoff aus den DOBAs, die nach den Vergleichsbeispielen 2 und 3 hergestellt worden sind. Es zeigt sich nämlich an den Ergebnissen aus Tabelle 1, dass das DOBA, welches nach den Vergleichsbeispielen 2 und 3 hergestellt worden ist, erst nach 18 Minuten 100 % der Perdecansäure generiert hat, wohingegen das DOBA, welches nach dem erfindungsgemäßen Beispiel 1 hergestellt worden ist, bereits nach 12 Minuten diesen Wert erreicht.

Das erfindungsgemäße Verfahren stellt somit im Hinblick auf den Einsatz der damit erhaltenen Acyloxybenzoesäuren der Formel (I) in Waschmitteln eine signifikante Verbesserung dar.

### Beispiel 9: Synthese von para-Decanoyloxy-benzoesäure (DOBA)

Das Verfahren entsprach Beispiel 1, jedoch wurde bei einer Ansatzgröße von 4500 mol die Temperatur während der Dosierung des Decansäurechlorids und des Nachrührens sowie bei der Zugabe der HCl-Lösung gemäß Schritt b) des erfindungsgemäßen Verfahrens auf 10 bis 15 °C gehalten. Die Reinheit des Produkts ist > 99,9 Gew.-%. Der d₁₀-Wert der Partikel ist 14,833 µm, der d₅₀-Wert ist 54,757 µm und der d₉₀-Wert ist 111,505 µm.

## Patentansprüche

1. Verfahren zur Herstellung von Acyloxybenzoesäuren der Formel (I) 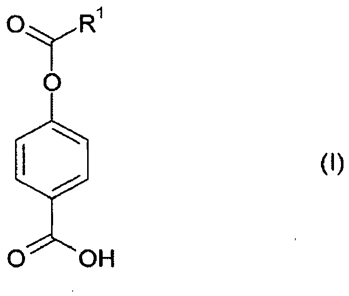 worin
R¹ eine lineare oder verzweigte, gesättigte Alkylgruppe mit 6 bis 30 Kohlenstoffatomen, eine lineare oder verzweigte, ein- oder mehrfach ungesättigte Alkenylgruppe mit 6 bis 30 Kohlenstoffatomen oder eine Arylgruppe mit 6 bis 30 Kohlenstoffatomen ist,
**dadurch gekennzeichnet, dass**
a) ein Carbonsäurehalogenid der Formel R¹COHal, worin R¹ die oben angegebene Bedeutung besitzt und Hal ein Halogenid ist, mit para-Hydroxybenzoesäure in Gegenwart von Base, wobei die eingesetzte Base ausgewählt ist aus Alkalihydroxiden, in einem Lösungsmittelgemisch enthaltend Wasser und ein organisches Lösungsmittel, wobei das eingesetzte organische Lösungsmittel iso-Propanol ist, bei einer Temperatur ≤ 25 °C und einem pH-Wert von 9 bis 11,5 umgesetzt wird,
b) das Reaktionsgemisch nach Schritt a) bei einer Temperatur ≤ 25 °C auf pH 6 bis 8 durch Zugabe von Säure eingestellt wird und
c) das Reaktionsgemisch nach Schritt b) auf eine Temperatur von 35 bis 80 °C erwärmt und danach durch Zugabe von Säure auf pH 1 bis 4 eingestellt wird.

2. Verfahren nach Anspruch 1, **dadurch gekennzeichnet, dass** nach Schritt c) das Reaktionsgemisch auf eine Temperatur < 35 °C abgekühlt wird (Schritt d)).

3. Verfahren nach Anspruch 1 oder 2, **dadurch gekennzeichnet, dass** die nach dem erfindungsgemäßen Verfahren hergestellten Acyloxybenzoesäuren der Formel (I) in Form von Partikeln mit d₅₀-Werten von 10 bis 150 µm vorliegen.

4. Verfahren nach Anspruch 3, **dadurch gekennzeichnet, dass** die Partikel d₁₀-Werte von 5 bis 30 µm und d₉₀-Werte von 30 bis 200 µm besitzen.

5. Verfahren nach einem oder mehreren der Ansprüche 1 bis 4, **dadurch gekennzeichnet, dass** die in Schritt a) eingesetzte Base KOH oder NaOH ist.

6. Verfahren nach einem oder mehreren der Ansprüche 1 bis 5, **dadurch gekennzeichnet, dass** die in Schritt b) und c) eingesetzte Säure einen pKa-Wert kleiner oder gleich 4,0 besitzt.

7. Verfahren nach einem oder mehreren der Ansprüche 1 bis 6, **dadurch gekennzeichnet, dass** die in Schritt b) und c) eingesetzte Säure H₂SO₄ oder HCl ist.

8. Verfahren nach einem oder mehreren der Ansprüche 1 bis 7, **dadurch gekennzeichnet, dass** das Gewichtsverhältnis von Wasser zu dem einen oder den mehreren organischen Lösungsmitteln in Schritt a) von 5 : 1 bis 1 : 5 ist.

9. Verfahren nach einem oder mehreren der Ansprüche 1 bis 8, **dadurch gekennzeichnet, dass** das Gewichtsverhältnis von Wasser zu para-Hydroxybenzoesäure in Schritt a) von 2 : 1 bis 10 : 1 ist.

## Claims

1. A process for preparing acyloxybenzoic acids of the formula (I) 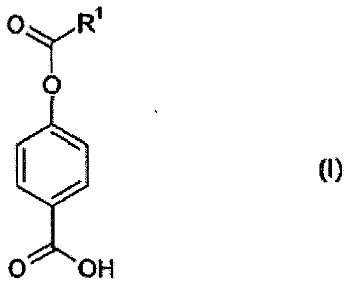 in which
R¹ is a linear or branched, saturated alkyl group having 6 to 30 carbon atoms, a linear or branched, singly or multiply unsaturated alkenyl group having 6 to 30 carbon atoms, or an aryl group having 6 to 30 carbon atoms,
**characterized in that**
a) a carboxylic halide of the formula R¹COHal, in which R¹ possesses the definition indicated above and Hal is a halide, is reacted with para-hydroxybenzoic acid in the presence of base, the base used being selected from alkali metal hydroxides, in a solvent mixture comprising water and an organic solvent, the organic solvent used being isopropanol, at a temperature ≤ 25°C and a pH of 9 to 11.5,
b) the reaction mixture after step a), at a temperature ≤ 25°C, is adjusted to a pH of 6 to 8 by addition of acid, and
c) the reaction mixture after step b) is heated to a temperature of 35 to 80°C and thereafter adjusted to a pH of 1 to 4 by addition of acid.

2. The process as claimed in claim 1, **characterized in that** after step c) the reaction mixture is cooled to a temperature < 35°C (step d)).

3. The process as claimed in claim 1 or 2, **characterized in that** the acyloxybenzoic acids of the formula (I) prepared by the process of the invention are present in the form of particles having d₅₀ values of 10 to 150 µm.

4. The process as claimed in claim 3, **characterized in that** the particles possess d₁₀ values of 5 to 30 µm and d₉₀ values of 30 to 200 µm.

5. The process as claimed in one or more of claims 1 to 4, **characterized in that** the base used in step a) is KOH or NaOH.

6. The process as claimed in one or more of claims 1 to 5, **characterized in that** the acid used in steps b) and c) possesses a pKa value of less than or equal to 4.0.

7. The process as claimed in one or more of claims 1 to 6, **characterized in that** the acid used in steps b) and c) is H₂SO₄ or HCl.

8. The process as claimed in one or more of claims 1 to 7, **characterized in that** the weight ratio of water to the one or more organic solvents in step a) is from 5:1 to 1:5.

9. The process as claimed in one or more of claims 1 to 8, **characterized in that** the weight ratio of water to para-hydroxybenzoic acid in step a) is from 2:1 to 10:1.

## Revendications

1. Procédé de préparation d'acides acyloxybenzoïques de formule (I) 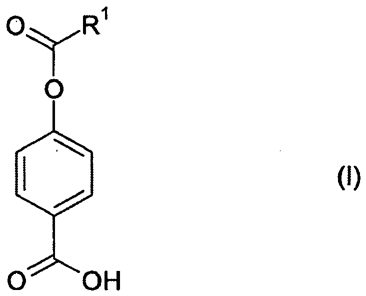 dans laquelle
R¹ est un groupe alkyle linéaire ou ramifié saturé, comprenant de 6 à 30 atomes de carbone, un groupe alcényle linéaire ou ramifié mono- ou polyinsaturé, comprenant de 6 à 30 atomes de carbone, ou un groupe aryle comprenant de 6 à 30 atomes de carbone,
**caractérisé en ce que**
a) un halogénure d'acide carboxylique de formule R¹COHal, dans laquelle R¹ possède la signification indiquée ci-dessus et Hal est un halogénure, est mis à réagir avec de l'acide para-hydroxybenzoïque en présence d'une base, la base utilisée étant choisie parmi les hydroxydes alcalins, dans un mélange de solvants contenant de l'eau et un solvant organique, le solvant organique utilisé étant de l'isopropanol, à une température ≤ 25 °C et un pH de 9 à 11,5,
b) le mélange de réaction selon l'étape a) à une température ≤ 25 °C est ajusté à un pH de 6 à 8 par ajout d'acide et
c) le mélange de réaction selon l'étape b) est chauffé à une température de 35 à 80 °C puis ajusté à un pH de 1 à 4 par ajout d'acide.

2. Procédé selon la revendication 1, **caractérisé en ce qu'**après l'étape c), le mélange de réaction est refroidi à une température < 35 °C (étape d)).

3. Procédé selon la revendication 1 ou 2, **caractérisé en ce que** les acides acyloxybenzoïques de formule (I) préparés d'après le procédé selon l'invention sont présents sous la forme de particules ayant des valeurs d₅₀ allant de 10 à 150 µm.

4. Procédé selon la revendication 3, **caractérisé en ce que** les particules possèdent des valeurs d₁₀ allant de 5 à 30 µm et des valeurs d₉₀ de 30 à 200 µm.

5. Procédé selon l'une ou plusieurs des revendications 1 à 4, **caractérisé en ce que** la base utilisée à l'étape a) est KOH ou NaOH.

6. Procédé selon l'une ou plusieurs des revendications 1 à 5, **caractérisé en ce que** l'acide utilisé aux étapes b) et c) possède un pKa inférieur ou égal à 4,0.

7. Procédé selon l'une ou plusieurs des revendications 1 à 6, **caractérisé en ce que** l'acide utilisé aux étapes b) et c) est H₂SO₄ ou HCl.

8. Procédé selon l'une ou plusieurs des revendications 1 à 7, **caractérisé en ce que** le rapport en poids entre l'eau et le ou les solvant(s) organique(s) à l'étape a) va de 5:1 à 1:5.

9. Procédé selon l'une ou plusieurs des revendications 1 à 8, **caractérisé en ce que** le rapport en poids entre l'eau et l'acide para-hydroxybenzoïque à l'étape a) va de 2:1 et 10:1.
